# EUROPÄISCHE PATENTSCHRIFT

(11) **EP 1 454 637 B1**
(45) Veröffentlichungstag und Bekanntmachung des Hinweises auf die Patenterteilung: **27.03.2013**
(21) Anmeldenummer: 04004195.6
(22) Anmeldetag: 25.02.2004
(51) Int. Cl.: A61L 2/24, A61L 2/18, C11D 11/00

(54) **Verfahren zum Reinigen und Desinfizieren medizinischer Instrumente**
Process for cleaning and disinfecting medical instruments
Procédé de nettoyage et désinfection d'instruments médicaux

(30) Priorität: 03.03.2003 DE 10309345
(43) Veröffentlichungstag der Anmeldung: 08.09.2004
(73) Patentinhaber: Miele & Cie. KG, 33332 Gütersloh (DE)
(72) Erfinder: Michels, Winfried, Dr., 34414 Warburg (DE); Pieper, Michael, 33378 Rheda-Wiedenbrück (DE)

(56) Entgegenhaltungen:
- EP-A- 0 268 227
- EP-A- 1 138 335
- EP-A- 1 260 234
- WO-A-01/47565
- DE-A- 3 327 466
- DE-A- 4 219 620
- DE-A- 4 342 573
- DE-A- 19 626 872
- ANTLOGA K ET AL: "PRION DISEASE AND MEDICAL DEVICES" ASAIO JOURNAL, J.B.LIPPINCOTT CO.,HAGERSTOWN,MD, US, Bd. 46, Nr. 6, 2000, Seiten S69-S72, XP001092854 ISSN: 1058-2916

## Beschreibung

Die Erfindung betrifft ein Verfahren zum Reinigen und Desinfizieren medizinischer Instrumente, bei dem mindestens die wasserführenden Programmabschnitte Vorspülen und Reinigen in einem Spülbehälter einer programmgesteuerten Spülmaschine durchgeführt werden, wobei beim Reinigen eine Flüssigkeit auf eine Temperatur zwischen 40°C und 60°C, vorzugsweise 55°C aufgeheizt wird, der als Behandlungsmittel ein alkalischer Reiniger derart zudosiert wird, dass sich ein pH-Wert zwischen 8,5 und 12 einstellt.

Aus der DE 43 42 573 A1 ist ein Verfahren zum Reinigen und thermischen Desinfizieren von Labor- und Operationsinstrumenten bekannt, das in einer programmgesteuerten Spülmaschine mit Spülbehälter durchgeführt wird. Das bekannte Verfahren dient zur Aufbereitung der Instrumente und umfasst die wasserführenden Spülprogramme Vorspülen, Reinigen, Neutralisation, Zwischenspülen und Thermodesinfektion, wobei die Spülprogrammabschnitte Vorspülen und Zwischenspülen fallweise einzeln oder in Kombination ausblendbar sind. Zur Austragung von besonders groben Blutanschmutzungen und zum Anlösen leicht angetrockneter Rückstände an den Instrumenten wird das Verfahren mit einem kurzen Vorspül-Programmabschnitt eingeleitet, welches mit kaltem, enthärtetem Wasser 1 bis 3 Minuten durchgeführt wird und welches mit dem Abpumpen der verschmutzten Flüssigkeit endet. Beim anschließenden Programmabschnitt Reinigen wird dem Spülbehälter zunächst enthärtetes Wasser und bis auf eine vorgegebene Temperatur zwischen 45°C und 60°C, vorzugsweise 55°C aufgeheizt. Während des Aufheizens wird der Reinigungsflüssigkeit ein alkalischer Reiniger zudosiert. Die Beschaffenheit des Reinigers und seine Dosiermenge sind so eingestellt, dass ein pH-Wert zwischen 8,5 und 12 erreicht wird. Damit der Reiniger seine optimale Wirkung entfaltet, wird die vorgegebene MaximalTemperatur für einige Minuten gehalten, bevor die Reinigungsflüssigkeit abgepumpt wird. Zur Neutralisation der Laugenrückstände auf dem Spülgut ist dann ein Programmabschnitt Neutralisieren nachgeschaltet, in dem die Instrumente mit einer mit einem sauren Neutralisator versetzten Flüssigkeit abgespült werden. Nach dem Neutralisationsschritt und abgepumpter Spülflüssigkeit folgt ein Programmabschnitt mit einem oder mehreren Zwischenspülgängen mit jeweils frischem Spülwasser, abhängig von der Verunreinigungen der Lauge und Instrumente. Nach erneutem Abpumpen werden die medizinischen Instrumente in einem Thermodesinfektions-Programmabschnitt mit auf 90°C aufgeheiztem Wasser behandelt, wobei vorzugsweise vollentsalztes Wasser (VE-Wasser) eingesetzt wird.

Bei einem solchen Verfahren zum Reinigen und Desinfizieren besteht das Problem, dass gerade schwer lösbare und auch schlecht sichtbare Rückstände, insbesondere Fibrinrückstände als vernetztes Blutbestandteil, auf dem aufzubereitenden Spülgut haften bleiben. Dies ist beispielsweise bei Koagulationsinstrumenten immanent. Im Hinblick auf eine Standardisierung der Reinigungsergebnisse auf hohem Niveau und als Voraussetzung für die Sicherheit der Sterilisation ist das bekannte Verfahren zur Instrumentenaufbereitung somit zu verbessern. Insbesondere ist dabei zu berücksichtigen, dass auch der Verhütung der Übertragung infektiöser Prionen, welche die Creutzfeldt-Jakob-Krankheit (CJK) oder deren neue Variante (vCJK) verursachen können und in den Fibrinrückständen eingebettet sein können bzw. aufgrund ihrer hohen Affinität zu Metall an der Oberfläche haften, sicher entgegengewirkt wird.

Es könnten die Fibrinrückstände bzw. Prionproteine möglicherweise dadurch entfernt werden, dass in einer extrem heißen Reinigungsflüssigkeit ein stark alkalischer Reiniger mit einem pH-Wert größer als 12 zugeführt wird. Jedoch wird durch diese Maßnahme der Werkstoff einiger zu reinigender Instrumente angegriffen. Hierbei erweisen sich einige Instrumentenwerkstoffe, beispielsweise optische Gläser und Aluminiuminstrumente als besonders empfindlich.

Aus der DE 38 43 992 C2 ist die Verwendung eines Flüssigkonzentrates zum Entfernen organischer Verschmutzungen bekannt. Dieses Konzentrat enthält als Aktivsauerstoffverbindung ein oder mehrere Peroxidschwefelsäuresalze. Eine solche hochmolekulare Verbindung enthält nur einen geringen Anteil von freien Sauerstoff-Radikalen, gemessen an ihrem Gesamt-Molekulargewicht. Um eine Menge von Aktivsauerstoff zu erhalten, die zum Reinigen und Desinfizieren medizinischer Instrumente ausreichen, müssten deshalb sehr große Mengen dieses Flüssigkonzentrates zudosiert werden. Angewendet in dem aus der DE 43 42 573 A1 bekannten Reinigungs-Programmabschnitt, würde die freien Sauerstoff-Radikale Verbindungen mit den in der Flüssigkeit gelösten Schmutzteilchen eingehen und deshalb nicht mehr zur Verfügung stehen, um die Anschmutzungen auf den Instrumenten zu lösen.

Die Verwendung von Peressigsäure ist zur Desinfektion bekannt. Die dabei entstehenden sauren Lösungen würden zur Denaturierung der Proteine führen und damit eine stärkere Verkrustung zur Folge haben.

Aus der WO01/47565 A2 ist ein Verfahren zur Reinigung und Desinfektion von thermolabilen medizinischen Geräten, wie Endoskopen bekannt, bei dem eine chemische Desinfektion mit einer wässrigen Desinfektionsmittellösung durchgeführt wird, welches Peressigsäure und/oder Wasserstoffperoxid enthält, wobei der pH-Wert der Desinfektionsmittellösung zwischen 5 und 9 liegt. Eine Reinigung mit einer alkalischen Reinigungslösung findet vor oder gleichzeitig mit der Desinfektion statt.

Ausgehend von dem eingangs beschriebenen Verfahren zur Reinigung und Desinfektion medizinischer Instrumente liegt der Erfindung die Aufgabe zugrunde, eine Optimierung der Instrumentenreinigung mit sicherer Beseitigung vernetzter, schwer löslicher und proteinhaltiger Anschmutzungen von der Instrumentenoberfläche bei materialschonenden Bedingungen zu schaffen sowie möglicherweise vorhandene Prionproteine zu zerstören.

Die Aufgabe wird dadurch gelöst, dass der Programmabschnitt Reinigen in zwei Teilabschnitte unterteilt ist, wobei zwischen dem ersten und dem zweiten Teilabschnitt ein Wechsel der Reinigungsflüssigkeit stattfindet, und wobei im zweiten Teilabschnitt der Flüssigkeit zusätzlich zum alkalischen Reiniger eine niedermolekulare Peroxidverbindung zudosiert wird. Bei einem pH-Wert über 8,5 zerfällt das niedermolekulare Peroxid spontan und spaltet sofort große Mengen Aktivsauerstoff ab. Da aufgrund des vorausgegangen Wechsels der Reinigungsflüssigkeit keine freien Schmutzteilchen mehr vorhanden sind, wirkt der Aktivsauerstoff in vorteilhafter Weise auf die hartnäckigen Anschmutzungen auf den Instrumenten ein; selbst die vCJK-Infektionen verursachenden Proteine werden oxidativ zerstört und in gut wasserlösliche Verbindungen überführt, die dann einfach mit der Reinigungsflüssigkeit weggespült werden können.

Dabei wird bei einer vorteilhaften Weiterentwicklung des Verfahrens ein tensidfreier Reiniger verwendet. Hierdurch wird eine unnötige Zehrung des. Aktivsauerstoffs verhindert.

Es ist außerdem vorteilhaft, wenn die niedermolekulare Peroxidverbindung nach dem alkalischen Reiniger der Flüssigkeit zudosiert wird. Hierdurch wird zunächst das alkalische Milieu geschaffen, welches zur Abspaltung der freien Sauerstoff-Radikale notwendig ist. Außerdem wird verhindert, dass ein neutrales oder saures Milieu entsteht, in welchem die Proteine denaturieren und damit stärker an den Instrumenten fixieren.

In einer zweckmäßigen Ausführungsform des Verfahrens zum Reinigen und Desinfizieren medizinischer Instrumente wird als niedermolekulare Peroxidverbindung eine Lösung von Peressigsäure oder von Wasserstoffperoxid verwendet.

Eine vorteilhafte Weiterentwicklung der erfindungsgemäßen Lehre sieht vor, dass die niedermolekulare Peroxidverbindung vor Erreichen der End-Temperatur der Flüssigkeit zudosiert wird. Da das Peroxid, sobald es in der Reinigungsflüssigkeit vorliegt, Sauerstoff abspaltet, verkürzt die vorgenannte Maßnahme die notwendige Dauer des Reinigungsgangs.

Nachfolgend ist das erfindungsgemäße Verfahren zum Reinigen und Desinfizieren medizinischer Instrumente, wie Labor- und Operationsinstrumente bspw. Zangen, Scheren, starre und ggf. flexible Endoskope, deren Optiken und dergleichen näher erläutert. Zu seiner Durchführung ist eine programmgesteuerte Spülmaschine vorgesehen. Diese weist zur Ablage des Instrumentariums, wie bekannt, einen Spülbehälter mit einem einschiebbaren Einsatzwagen auf. Grundsätzlich kann das nachfolgend beschriebene erfindungsgemäße Verfahren auch in Programmabläufe von Spülautomaten implementiert werden, die zum Reinigen, Spülen und thermischen Desinfizieren von in der orthopädischen Chirurgie und/oder Zahnmedizin eingesetzten Motoren- und Antriebssystemen vorgesehen sind.

Die Spülmaschine zur Durchführung des erfindungsgemäßen Verfahrens benutzt zum Reinigen und zur Desinfektion auf vorgebbare Behandlungstemperaturen (Reinigungs- Desinfektionstemperatur) aufheizbare Reinigungsflüssigkeiten, denen bedarfsweise Reinigungsmittel zugemischt werden. Dem Spülbehälter der Spülmaschine wird die Reinigungsflüssigkeit als kaltes oder erwärmtes, weiches Brauchwasser bzw. Frischwasser zugeführt, welches ggf. auch enthärtetes Weichwasser sein kann. Der Spülautomat weist außerdem einen Anschluss für vollentsalztes Wasser (VE-Wasser) auf. Die in den Spülbehälter eingelassene Reinigungsflüssigkeit wird in an sich bekannter Weise in wasserführenden Programmabschnitten umgewälzt und auf die zu reinigenden und anschließend zu desinfizierenden Instrumente gesprüht.

Das erfindungsgemäße Verfahren zum Reinigen und Desinfizieren umfasst die nacheinander ablaufenden wasserführenden Programmabschnitte Vorspülen, Reinigen, Neutralisation, Zwischenspülen und die thermische Desinfektion, wobei Zwischenspülgänge von der Programmsteuerung auch mehrfach nacheinander aufgerufen werden können. Ferner kann zur Wasser- und Energieeinsparung vorgesehen sein, das Zwischenspülen abhängig vom jeweiligen Verschmutzungsgrad der zu reinigenden Gerätschaften auszublenden.

Zum Abspülen von groben Anschmutzungen am Instrumentarium und leicht entfernbaren Blutmengen wird als erster Verfahrensschritt der Programmabschnitt Vorspülen ausgeführt, bei dem kaltes, enthärtetes Wasser in den Spülraum der Spülmaschine geleitet wird. Das Aufheizen unterbleibt, da die zu entfernenden Anschmutzungen bei niedrigen Temperaturen weniger aufschäumen. Nach dem 1 bis 3 Minuten dauernden Vorspülen schließt sich nach einem Wasserwechsel ein erster beheizter Reinigungsgang an, bei dem die Reinigungsflüssigkeit bis auf eine End-Temperatur von 55°C erhitzt wird. Beim Erreichen einer Temperatur von etwa 40°C wird als Behandlungsmittel ein alkalischer Reiniger automatisch zudosiert. Die Menge des Reinigers und seine Alkalität ist derart bemessen, dass ein optimaler pH-Wert von 11 bis 11,5 zum Lösen der an dem Spülgut anhaftenden Anschmutzungen realisiert wird. Der Reiniger enthält vorzugsweise ein Silikat zum Schutz der aufzubereitenden Materialien und ist außerdem tensidfrei. Nach dem Erreichen der End-Temperatur von 55°C wird diese für 5 bis 10 Minuten gehalten und der Reinigungsgang so lange fortgesetzt. Anschließend wird die Reinigungsflotte entleert.

Danach wird dem Spülraum wieder enthärtetes Wasser zugeführt und erhitzt. Außerdem erfolgt erneut bei 40°C die Zudosierung des alkalischen Reinigers und die damit verbundene Einstellung des pH-Werts auf 11 bis 11,5. Nach dem Zudosieren des alkalischen Reinigers wird der Reinigungsflüssigkeit zusätzlich eine niedermolekulare Peroxidverbindung beigegeben.

Die Einstellung eines pH-Werts auf einen Wert zwischen 8,5 und 12 durch einen alkalischen Reiniger bewirkt, dass das Peroxid in der Reinigungsflüssigkeit spontan zerfällt und sofort ein hohes Oxidationspotential bereitstellt. Durch eine solche Oxidation werden die hartnäckig und schwer lösbaren Anschmutzungen wie Fibrinrückstände sowie Koagulationen an Instrumenten der Hochfrequenzchirurgie, die in nicht sichtbaren Belägen am Material des Spülgutes haften bleiben, zerstört bzw. inaktiviert und in gut wasserlösliche Verbindungen überführt. Um besonders viele solcher Anschmutzungen in kurzer Zeit zu inaktivieren und in gut wasserlösliche Verbindungen zu überführen, wird die Reinigungsflüssigkeit wieder bis auf eine End-Temperatur von 55 °C aufgeheizt. Diese Temperatur ist niedrig genug, um ein Denaturieren der proteinhaltigen Rückstände zu vermeiden und eine schnelle Sauerstoffabspaltung vom Peroxid zu gewährleisten. Damit alle proteinhaltigen Anschmutzungen sicher zerstört werden und vom Spülgut abgelöst werden, wird die End-Temperatur bis zu 10 Minuten (konstant) gehalten, bevor die Reinigungsflüssigkeit mit den von den Instrumenten abgelösten hartnäckigen Anschmutzungen abgepumpt wird.

Als Behandlungsmittel eignen sich besonders Wasserstoffperoxid oder eine Lösung von Peressigsäure, da sie niedermolekular sind und damit einen hohen Anteil ihrer Gesamtmasse als Aktivsauerstoff freisetzen. Andererseits sind sie handelsüblich und preisgünstig. Wasserstoffperoxid ist zu bevorzugen, da aufgrund seiner Neutralität der durch den alkalischen Reiniger eingestellte pH-Bereich der Reinigungsflüssigkeit nicht verschoben wird, was bei der Verwendung von Peressigsäure der Fall wäre. Ein weiterer Vorteil von Wasserstoffperoxid ist der, dass es nur in Wasser und Sauerstoff zerfällt und die verbleibenden Rückstände deshalb keiner Risikoanalyse bedürfen.

Nach Beendigung des letzten Reinigungsschritts werden die an sich bekannten und deshalb nachfolgend nicht beschriebenen Verfahrensschritte Neutralisation und thermische Desinfektion aufgerufen.

## Patentansprüche

1. Verfahren zum Reinigen und Desinfizieren medizinischer Instrumente, bei dem mindestens die wasserführenden Programmabschnitte Vorspülen, Reinigen Neutralisation und thermische Desinfektion in einem Spülbehälter einer programmgesteuerten Spülmaschine durchgeführt werden, wobei beim Reinigen eine Flüssigkeit auf eine Temperatur zwischen 40°C und 60°C, vorzugsweise 55°C aufgeheizt wird, der als Behandlungsmittel ein alkalischer Reiniger derart zudosiert wird, dass sich ein pH-Wert zwischen 8,5 und 12 einstellt,
**dadurch gekennzeichnet,**
**dass** der Programmabschnitt Reinigen in zwei Teilabschnitte unterteilt ist, wobei der Reinigungsflüssigkeit im ersten Teilabschnitt ein alkalischer Reiniger zudosiert wird und wobei zwischen dem ersten und dem zweiten Teilabschnitt ein Wechsel der Reinigungsflüssigkeit stattfindet, wobei im zweiten Teilabschnitt ein alkalischer Reiniger zudosiert wird, und im zweiten Teilabschnitt zusätzlich zum alkalischen Reiniger eine niedermolekulare Peroxidverbindung zudosiert wird, und dass nach Beendigung des letzten Reinigungsschrittes die Programmabschnitter Neutralisation und thermische Desinfektion durchgeführt werden.

2. Verfahren zum Reinigen und Desinfizieren medizinischer Instrumente nach Anspruch 1,
**dadurch gekennzeichnet,**
**dass** als alkalischer Reiniger ein tensidfreier Reiniger verwendet wird.

3. Verfahren zum Reinigen und Desinfizieren von medizinischer Instrumente nach Anspruch 1 oder 2,
**dadurch gekennzeichnet,**
**dass** die niedermolekulare Peroxidverbindung nach dem alkalischen Reiniger der Flüssigkeit zudosiert wird.

4. Verfahren zum Reinigen und Desinfizieren von medizinischer Instrumente nach mindestens einem der Ansprüche 1 bis 3,
**dadurch gekennzeichnet,**
**dass** als niedermolekulare Peroxidverbindung eine Lösung von Peressigsäure oder von Wasserstoffperoxid verwendet wird.

5. Verfahren zum Reinigen und Desinfizieren medizinischer Instrumente nach mindestens einem der Ansprüche 1 bis 4,
**dadurch gekennzeichnet,**
**dass** die niedermolekulare Peroxidverbindung vor Erreichen der End-Temperatur der Flüssigkeit zudosiert wird.

6. Verfahren zum Reinigen und Desinfizieren medizinischer Instrumente nach mindestens einem der Ansprüche 1 bis 5,
**dadurch gekennzeichnet,**
**dass** die End-Temperatur der mit dem alkalischen Reiniger und mit der niedermolekularen Peroxidverbindung versetzten Flüssigkeit mindestens 5 Minuten gehalten wird.

## Claims

1. Method for cleaning and disinfecting medical instruments, in which at least the rinsing, cleaning, neutralisation and thermal disinfection water-conducting program portions are carried out in a washing container of a program-controlled dishwasher, a liquid being heated to a temperature of between 40 °C and 60 °C, preferably of 55 °C, during cleaning, to which liquid an alkali cleaner is added as a treatment agent in such a way that a pH value of between 8.5 and 12 is set,
**characterised in that**
the cleaning program portion is divided into two sub-portions, an alkali cleaner being added to the cleaning liquid in the first sub-portion and a change of cleaning liquid taking place between the first and the second sub-portion, an alkali cleaner being added in the second sub-portion and a low-molecular peroxide compound being added in the second sub-portion in addition to the alkali cleaner, and **in that** the neutralisation and thermal disinfection program portions are carried out upon termination of the final cleaning step.

2. Method for cleaning and disinfecting medical instruments according to claim 1,
**characterised in that**
a surfactant-free cleaner is used as the alkali cleaner.

3. Method for cleaning and disinfecting medical instruments according to either claim 1 or claim 2,
**characterised in that**
the low-molecular peroxide compound is added to the liquid after the alkali cleaner.

4. Method for cleaning and disinfecting medical instruments according to at least one of claims 1 to 3,
**characterised in that**
a peracetic acid solution or hydrogen peroxide solution is used as the low-molecular peroxide compound.

5. Method for cleaning and disinfecting medical instruments according to at least one of claims 1 to 4,
**characterised in that**
the low-molecular peroxide compound is added to the liquid before the final temperature is reached.

6. Method for cleaning and disinfecting medical instruments according to at least one of claims 1 to 5,
**characterised in that**
the final temperature of the liquid which has been mixed with the alkali cleaner and with the low-molecular peroxide compound is maintained for at least 5 minutes.

## Revendications

1. Procédé de nettoyage et de désinfection d'instruments médicaux, dans lequel au moins les segments de programme véhiculant de l'eau que sont le prélavage, le nettoyage, la neutralisation et la désinfection thermique sont effectués dans une cuve de lavage d'une machine de lavage commandée par programme, un liquide étant, lors du nettoyage, chauffé à une température entre 40° C et 60° C, de préférence à 55° C, liquide auquel est ajouté un nettoyant alcalin en tant que moyen de traitement de telle sorte qu'il s'établit un pH entre 8,5 et 12,
**caractérisé en ce que**
le segment de programme nettoyage est divisé en deux segments partiels, un nettoyant alcalin étant ajouté au liquide de nettoyage dans le premier segment partiel et un remplacement du liquide de nettoyage intervenant entre le premier segment partiel et le deuxième segment partiel, un nettoyant alcalin étant également ajouté dans le deuxième segment partiel, et un composé peroxydé à faible masse moléculaire étant ajouté au nettoyant alcalin dans le deuxième segment partiel, et **en ce que**, une fois achevée la dernière étape de nettoyage, les segments de programme neutralisation et désinfection thermique sont effectués.

2. Procédé de nettoyage et de désinfection d'instruments médicaux selon la revendication 1,
**caractérisé en ce que**,
en tant que nettoyant alcalin, il est utilisé un nettoyant exempt d'agent tensio-actif.

3. Procédé de nettoyage et de désinfection d'instruments médicaux selon la revendication 1 ou 2,
**caractérisé en ce que**
le composé peroxydé à faible masse moléculaire est ajouté au liquide après le nettoyant alcalin.

4. Procédé de nettoyage et de désinfection d'instruments médicaux selon au moins une des revendications 1 à 3,
**caractérisé en ce que**,
en tant que composé peroxydé à faible masse moléculaire, il est utilisé une solution d'acide peracétique ou de peroxyde d'hydrogène.

5. Procédé de nettoyage et de désinfection d'instruments médicaux selon au moins une des revendications 1 à 4,
**caractérisé en ce que**
le composé peroxydé à faible masse moléculaire est ajouté au liquide avant l'obtention de la température finale.

6. Procédé de nettoyage et de désinfection d'instruments médicaux selon au moins une des revendications 1 à 5,
**caractérisé en ce que**
la température finale du liquide qui a été mélangé avec le nettoyant alcalin et le composé peroxydé à faible masse moléculaire est maintenue pendant au moins 5 minutes.
